(19) Europäisches Patentamt
European Patent Office
Office européen des brevets

(11) **EP 4 632 379 A1**

(12) **EUROPEAN PATENT APPLICATION**

(43) Date of publication:
**15.10.2025 Bulletin 2025/42**

(21) Application number: **25167781.1**

(22) Date of filing: **01.04.2025**

(51) International Patent Classification (IPC):
*G01N 33/50* (2006.01)  *G01N 33/58* (2006.01)
*C12Q 1/04* (2006.01)  *C12Q 1/6816* (2018.01)
*C12Q 1/6841* (2018.01)

(52) Cooperative Patent Classification (CPC):
**C12Q 1/04; C12Q 1/6816; C12Q 1/6841;
G01N 33/5091; G01N 33/588;** C12Q 2563/107;
C12Q 2565/607

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **01.04.2024 PT 2024119356
28.10.2024 PT 2024119794**

(71) Applicant: **Eurolustre Fábrica de Candeeiros Lda
4430-286 Vila Nova de Gaia (PT)**

(72) Inventors:
• **BESSA DE OLIVEIRA, JOSÉ MANUEL
MATOSINHOS (PT)**
• **CORREIA DE OLIVEIRA, PEDRO MIGUEL
VILA REAL (PT)**

(74) Representative: **Pereira da Cruz, Joao
J. Pereira da Cruz, S.A.
Rua Victor Cordon, 10-A
1249-103 Lisboa (PT)**

Remarks:
The complete document including Reference
Table(s) and the Sequence Listing(s) can be
downloaded from the EPO website

(54) **OPTICAL SYSTEM FOR FLUORESCENCE-BASED GENETIC DIAGNOSIS**

(57)    The present disclosure presents a system for fluorescence-based detection and quantification of a biomolecule in a sample, the system comprising three distinct units: a first unit comprising a heating unit with at least one integrated heat probe and a data integration system, such as a computer or an exterior computer; a second unit comprising at least one optical unit, preferably a spectrometer, which is connected to the computer or exterior computer of the first unit and means for receiving a sample containing the biomolecule to be analysed; and a third unit comprising a timer and a temperature control unit. It is also encompassed a process for use of the system and its use in medicine, preferably in *in-vitro* cancer detection and monitoring, infectious disease diagnosis, genetic disorders screening, pharmacogenomics for personalized medicine and neurological disorder diagnosis.

EP 4 632 379 A1

**Description**

**FIELD OF THE INVENTION**

The present disclosure relates to the field of Biotechnology and Medicine. It relates to screening devices, particularly to the field of detection system for identification and quantification of biomolecules using fluorescence-based diagnostic kits.

**PRIOR ART**

Fluorescence-based diagnostic techniques leverage the fluorescence properties of certain molecules to detect and analyze various substances or conditions. Some common techniques used in state-of-the-art fluorescence-based diagnostic system are the following:

Fluorescence Microscopy: This technique involves illuminating a sample with a specific wavelength of light, causing fluorescent molecules within the sample to emit light of a longer wavelength. It's widely used in medical diagnostics, microbiology, and cell biology for visualizing and analyzing cellular structures, proteins, and other biomolecules.

Fluorescence Spectroscopy: Fluorescence spectroscopy measures the intensity and characteristics of fluorescent light emitted by a sample when excited by a specific wavelength of light. It's used for qualitative and quantitative analysis of various substances, such as biomarkers, drugs, pollutants, and environmental compounds.

Flow Cytometry: Flow cytometry utilizes fluorescence to analyze and sort individual cells or particles in a fluid suspension. It can provide information about cell size, granularity, and fluorescence intensity, making it valuable in immunology, oncology, and hematology for cell counting, cell sorting, and biomarker analysis.

Fluorescence *In Situ* Hybridization (FISH): FISH is a molecular cytogenetic technique that uses fluorescent organic probes to detect and localize specific DNA sequences within chromosomes or cells. It's used for identifying genetic abnormalities, studying gene expression, and diagnosing genetic diseases and cancers.

Fluorescence Resonance Energy Transfer (FRET): FRET measures the transfer of energy between two fluorophores (donor and acceptor) when they are in close proximity. It's employed in molecular biology and biochemistry to study protein-protein interactions, protein conformational changes, and nucleic acid dynamics.

Fluorescence Lifetime Imaging Microscopy (FLIM): FLIM measures the lifetime of fluorescence emission in addition to intensity. It's used to investigate molecular interactions, protein dynamics, and metabolic processes within cells and tissues. FLIM can provide valuable insights into cellular function and disease pathology.

Fluorescence-Based Immunoassays: These assays utilize fluorescently labeled antibodies or antigens to detect and quantify specific proteins or biomarkers in biological samples. They are widely used in clinical diagnostics for detecting infectious diseases, autoimmune disorders, and cancer biomarkers with high sensitivity and specificity.

Quantitative Polymerase Chain Reaction (qPCR): qPCR combines fluorescence detection with the polymerase chain reaction to amplify and quantify specific DNA sequences. It's used for gene expression analysis, pathogen detection, and genetic testing in research, clinical diagnostics, and forensic science.

These techniques are continually evolving with advancements in fluorescence probes, instrumentation, and data analysis methods, enabling more sensitive, specific, and multiplexed diagnostic applications in various fields of science and medicine. However, all the existing solutions have the issue of requiring external system in order to successfully detect the analyte, e.g., thermoblocks. These external systems enhance the overall measurement complexity and usually require specialized personnel to operate it. Moreover, since these systems are usually bulky and do not have a power supply, they are not adequate for *in situ* measurements, i.e., they lack portability.

Most fluorescence-based detectors also require an external computer, to insert data and operate the system.

Solutions exist in the art where, it is possible to use smartphones for some fluorescence measurements and small devices that enable portability; however, they still require additional system to perform a successful analysis. The analysis time is often more than a minute.

Moreover, the fluorescence detection is often only possible using highly fluorescent molecules/nanoparticles, e.g., Green Fluorescent Protein, Rhodamine, Fluorescein and their combination with metal and lipid nanoparticles. The use of Carbon nanoparticles, for example, that usually have a low quantum yield, is not possible.

The devices of the state of the art are only suitable for highly fluorescence markers and this has the major drawback of lack of versatility towards the low quantum yield fluorophores being produced. In fact, when the high fluorescence sensitivity is an issue, there is, at present, no portable device that enables a reliable measurement.

The devices of the state of the art are also not user-friendly and versatile in terms of sample preparation, particularly when it is necessary to carry out sample pre-treatment steps. They are not adjustable in order to allow the sample pre-treatment and analysis to be performed in the same system, using different components already integrated and with independent controls.

These facts are disclosed in order to illustrate the technical problem addressed by the present disclosure.

The present solution intended to innovatively overcome such issues.

## GENERAL DESCRIPTION

In a preferred embodiment, the present disclosure relates to a system for fluorescence-based detection and quantification of a biomolecule in a sample, the system comprising three distinct units: a first unit comprising a heating unit with at least one integrated heat probe and a data integration system, such as a computer or an exterior computer; a second unit comprising at least one optical unit, preferably a spectrometer, which is connected to the computer or exterior computer of the first unit and means for receiving a sample containing the biomolecule to be analysed; and a third unit comprising a timer and a temperature control unit. A heat probe in optical devices refers to an instrument that uses light-based techniques to monitor, map, or influence temperature variations or thermal properties within a material or system. It typically involves detecting emitted infrared radiation, measuring changes in optical properties due to temperature shifts, or using focused laser beams to induce localized heating.

In a further embodiment, it is disclosed a system comprising an oligonucleotide chosen from: SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9 and/, SEQ ID No. 10 SEQ ID No. 11, or SEQ ID No. 12.

In a further embodiment, it is disclosed a system, wherein said units comprise primarily iron, about 18-20% chromium and 8-10.5% (w/w) nickel and optionally small amounts, particularly $\leq$ 2.0% (w/w), of manganese, silicon, carbon and/or phosphorus. This particular composition applied to this particular system provides high resistance to corrosion and oxidation, particularly in atmospheric and mild chemical environments, high ductility, strength, and toughness, even at low temperatures, generally nonmagnetic in the annealed condition but can become slightly magnetic when cold-worked, excellent weldability, with no need for post-weld annealing.

In a further embodiment, it is disclosed a system further comprising an external power source, such as an electrical power supply unit, a battery module, a solar power unit, a USB power interface and/or a fuel cell unit, wherein the external power source system is configured to selectively or concurrently provide power from one or more of the power units to the system based on power availability, environmental conditions, or user preference.

In a further embodiment, it is disclosed a system further comprising an excitation source comprising a wavelength from 400 to 850 nm.

In a further embodiment, it is disclosed a system further comprising an excitation source, such as a LED, a laser, a xenon lamp, a mercury vapor lamp and/or a halogen lamp.

In a further embodiment, it is disclosed a system further comprising means for centrifugation, particularly suitable to centrifugate samples at a maximum speed from 6000 rpm to 12000 rpm, which is incorporated inside the system or used externally.

In a further embodiment, it is disclosed a system further comprising means for pre-treatment of the sample, preferably centrifugation means and/or agitation means, such as vortex.

In a further embodiment, it is disclosed a system further comprising at least one optical fiber with quartz core, a sampler unit, a transistor for LED, a pressure switch for the heating unit

In a further embodiment, it is disclosed a system enclosed in a case, such as a box.

In a further embodiment, it is disclosed a process for *in vitro* detecting a given biomolecule in the system of the present disclosure comprising the steps of:

    recognizing a given fluorescent-based diagnostic kit;
    collecting a sample, preferably a nasopharyngeal or oropharyngeal swab, serum, blood, faeces or a tissue extract;
    optionally pre-treating said sample;
    placing the sample in a vial, preferably a tube, comprising an inactivation solution;
    assigning a code bar to said sample;
    passing said sample through the bar code reader or QR code identification unit;
    placing said sample, preferably 5 $\mu$l, into one to three vials/cuvettes;
    detecting said biomolecule in said sample by identifying a target genetic sequence.

In a particular embodiment, it is disclosed the use of the system of the present disclosure in medicine, preferably in *in-vitro* cancer detection and monitoring, infectious disease diagnosis, genetic disorders screening, pharmacogenomics for personalized medicine and neurological disorder diagnosis.

In a particular embodiment, it is disclosed the use of the system of the present disclosure in *in-vitro* detection of *Eutypa lata,* HEV or SARS-CoV-2 infection.

The present application relates to a novel detection system for genetic diagnostic/screening. The detection is fluorescence-based, meaning any technique, assay, or method that relies on fluorescence-the process where certain molecules absorb light and then emit light at a longer wavelength as they return to their normal state.

The object of the present disclosure emerges from the pressing need for a versatile, specific and low-cost solution that can be used for genetic fluorescence-based diagnostic in both laboratory conditions, as well as, in point-of-care (POC) applications.

In an embodiment, the system of the present application is used for detection of any biological molecule which is detectable by means of any diagnostic kit.

For "biological molecule", the following are encompassed:

Carbohydrates: Carbohydrates are organic compounds composed of carbon, hydrogen, and oxygen atoms. They serve as a primary source of energy and play structural roles in cells. Examples include glucose, sucrose, cellulose, and glycogen.

Lipids: Lipids are hydrophobic molecules that include fats, oils, phospholipids, and steroids. They serve as energy storage molecules, components of cell membranes, and signalling molecules.

Examples include triglycerides, phospholipids, cholesterol, and hormones such as estrogen and testosterone.

Proteins: Proteins are large, complex molecules composed of amino acids linked by peptide bonds. They perform a wide range of functions in cells, including enzymatic catalysis, structural support, transport, signalling, and immune defence. Examples include enzymes, antibodies, haemoglobin, and collagen.

Nucleic Acids: Nucleic acids are macromolecules that store and transmit genetic information. There are two main types: deoxyribonucleic acid (DNA) and ribonucleic acid (RNA). DNA carries the genetic instructions for the development, functioning, growth, and reproduction of organisms, while RNA plays roles in protein synthesis and gene regulation.

Vitamins: Vitamins are organic compounds that are essential for various metabolic processes in organisms. They often function as coenzymes or cofactors in enzymatic reactions. Examples include vitamin C (ascorbic acid), vitamin D (calciferol), and the various B vitamins (e.g., B12, folate).

Hormones: Hormones are signalling molecules produced by glands in multicellular organisms. They regulate physiological processes such as growth, metabolism, reproduction, and response to stress.

Examples include insulin, adrenaline, estrogen, and testosterone.

Metabolites: Metabolites are small molecules involved in metabolic pathways within cells. They include intermediates and end products of metabolism, such as ATP (adenosine triphosphate), NADH (nicotinamide adenine dinucleotide), and acetyl-CoA.

Immunological markers: Immunological biomarkers are biological markers for cellular activation and play an important role in the immune system function. They include, but are not limited to, serum cytokines, chemokines, adipocytokines, soluble forms of cell receptors, and immune activation markers.

In an embodiment, the system operational procedure comprises the following steps:

A given diagnostic kit identification code is passed on the code bar reader/QR code camera of the system of the present disclosure so that a specific software initiates a protocol designed and previously inserted into the computer unit of the system;

If it is necessary to use a heating chamber, the protocol designed will activate it and signal when it is ready for measurement; the heating chamber is advantageous when the biological sample comprises cells and it is necessary to promote the cell membrane disruption and the inactivation of biological enzymes that can interfere with the result;

Each testing sample is assigned a code bar. The end-user will pass the sample through the bar code reader/QR code camera before performing the analysis; in an embodiment the sample is a nasopharyngeal or oropharyngeal swab, serum, blood, faeces or tissue extracts.

The cuvette with the diagnostic kit is placed inside the reading chamber;

A message appears in the LCD signalling the measurement of the sample (this takes approximately 300 ms); when ready to change the cuvette another message appears.

Finally, the result appears in the form of a light or message indicating the result, which can be positive, negative or inconclusive;

The data are recorded automatically and the end-user has the opportunity to select if the result will be sent directly to the patient or not.

In one embodiment, the system of the present disclosure comprises a heating unit that is used independently of the optical unit and that has a fast and stable heating ramp.

In one embodiment, the system has a QR code identification unit and a bar code reader which allows the insertion of the diagnostic kit identification and also the patient information.

In one embodiment the system comprises an integrated computer and optionally a display unit.

In one embodiment the system has an external power source that allows its application outside laboratory conditions (for example in point-of-care units - POC).

In yet another embodiment, the system comprises an excitation source with variable wavelengths, according to each diagnostic kit specifications, ranging from 400 to 850 nm.

The system of the present disclosure comprises at least one optical unit which is responsible for the fluorescence excitation and emission collection.

The present application relates to fluorescence-based detection system for detecting and quantifying the presence of different biomolecules, particularly nucleic acids.

This diagnostic system of the present disclosure provides an ultrasensitive, robust, rapid and low-cost tool for detection of different biomolecules, which is adaptable to several diagnostic kits for detecting biomolecules.

The technology herein described is differs comprises the following advantages:

It has its own power source so it can be used in point-of-care (POC) applications;

It can be used by non-specialized personnel;

It is to be used with diagnostic kits which are fluorescence-based and its optical components can be adapted so different wavelength specifications can be used;

It can have more than one optical unit that allows 1-3 tests to be performed simultaneously;

The computer and display unit allow the insertion of data and reading/exporting/printing or sending the results directly to the patient.

The heating unit which can be used separately from the system or as part of the measurement procedure is very efficient and allows the stabilization of the chamber in temperatures ranging from 40-500ºC with variations of approximately 1ºC.

It can be used to detect and quantify in one analysis, depending on the diagnostic kit used and the insertion of the calibration data on the system software.

In an embodiment, the heating unit is independent, thus it can be used independently of the optical system. If, for example, it is necessary to heat a sample *in situ* but that sample will not be used for the diagnostic kit, thus it will not require the optical unit, it is possible to use the heating unit for that purpose.

The system of the present disclosure is also time efficient. The optical unit reads fluorescence signals and interpret the results in less than a second.

In an embodiment, the present system comprises the insertion of at least one pre-treatment unit. In the context of the present disclosure, pre-treatment shall be intended as centrifugation, temperature variation, mixing, among other necessary pre-treatment steps needed according to the sample matrix (faeces, blood, saliva, swabs, etc).

In a preferred embodiment, the disclosure relates to a system for fluorescence-based detection and quantification of a biomolecule in a sample, the system comprising at least one optical unit, a heating unit which is independent from the optical unit, a QR code identification unit, a bar code reader, an integrated computer, preferably a mini-pc, a timer, a software, a display unit, an external power source, an excitation source comprising a wavelength from 400 to 850 nm and means for receiving a fluorescent-based diagnostic kit.

In a further embodiment the disclosure relates to a system comprising at least two or three optical units.

In a further embodiment the disclosure relates to a system further comprising means for pre-treatment of the sample, preferably centrifugation means and/or agitation means, such as vortex.

In a further embodiment the disclosure relates to a system wherein the excitation source is a LED.

In an embodiment, the present disclosure relates to a process for *in vitro* detecting a given biomolecule in the system of the present disclosure comprising the steps of:

Recognizing a given fluorescent-based diagnostic kit;

collecting a sample, preferably a nasopharyngeal or oropharyngeal swab, serum, blood, faeces or a tissue extract;

optionally pre-treating said sample;

placing the sample in a vial, preferably a tube, comprising an inactivation solution;

assigning a code bar to said sample;

passing said sample through the bar code reader or QR code identification unit;

placing said sample, preferably 5$\mu$l, into one to three vials/cuvettes;

detecting said biomolecule in said sample.

In a further embodiment, the system also comprises an external power source that is able to provide at least one-hour operating ability without being directly plugged into electricity. It also has an integrated QR code camera and a bar code reader.

The features sample holder, optical unit and light emitter can be duplicated, triplicated until the multiplication needed and suitable to be accommodated by the device of the present disclosure. The increasing of these units within the system provides the ability to carry multiple tests at the same time.

For the purpose of interpretation of the present invention, "inactivation solution" shall be interpreted as a solution that is used for stabilizing biological samples from the collection place until its arrival at the laboratory and/or is responsible for disrupting the viral membrane, thus protecting the analysing technician as it turns the procedure a safer one when compared with other procedures.

In an embodiment, the present disclosure relates to the use of the system of the present disclosure in medicine, preferably in cancer detection and monitoring, infectious disease diagnosis, genetic disorders screening, pharmacogenomics for personalized medicine and neurological disorder diagnosis.

## DESCRIPTION OF FIGURES

The following figures provide preferred embodiments for illustrating the disclosure and should not be seen as limiting the scope of invention.

Figure 1 represents the layout of the Interior supporting unit (A) and the external System of the present disclosure box (B).

Figure 2 represents the configuration of the Interior supporting unit (A), with the several components identified below. AISI 304 is a type of stainless steel commonly used for various components and applications due to its excellent corrosion resistance, durability, and good mechanical properties.

### Description of the interior supporting units

| Name | Dimensions | Quality | Weight/ kg |
|---|---|---|---|
| P0001 | 235x160 | Aisi 304 | 0.424 |
| P0002 | 242x170 | Aisi 304 | 0.418 |
| P0003 | 242x104 | Aisi 304 | 0.226 |
| P0004 | 164x15 | Aisi 304 | 0.019 |
| P0005 | 101x75 | Aisi 304 | 0.056 |
| P0006 | 103x74 | Aisi 304 | 0.025 |
| P0007 | 50x15 | Aisi 304 | 0.007 |
| P0008 | 29x24 | Aisi 304 | 0.004 |
| P0009 | 16x16 | Aisi 304 | 0.008 |
| P0010 | 16x4 | Aisi 304 | 0.002 |

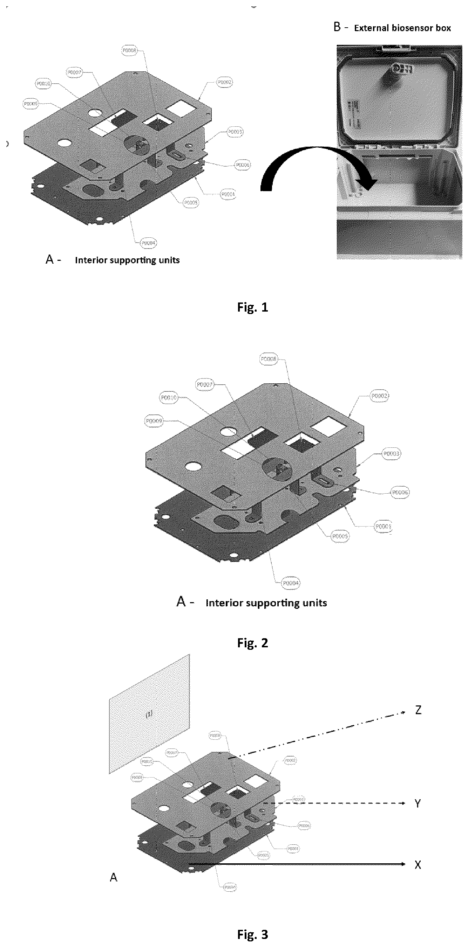

Figure 3 is a configuration of the Interior supporting unit (A) comprising three levels of components: first level X, second level Y and third level Z, each level being characterized below:

| 1st Level X | 2nd Level Y | 3rd Level Z |
|---|---|---|
| Bulk hardware: | Bulk hardware: | Bulk hardware: |
| ➢ Heating unit with integrated heat probe; | ➢ Spectrometer connected to the mini computer or to a portable exterior PC | ➢ Timmer unit; |
| ➢ Mini computer unit connected to the tablet screen on the (1) and the spectrometer on the 2nd level | ➢ Cuvette sampler. | ➢ Heat controller unit |

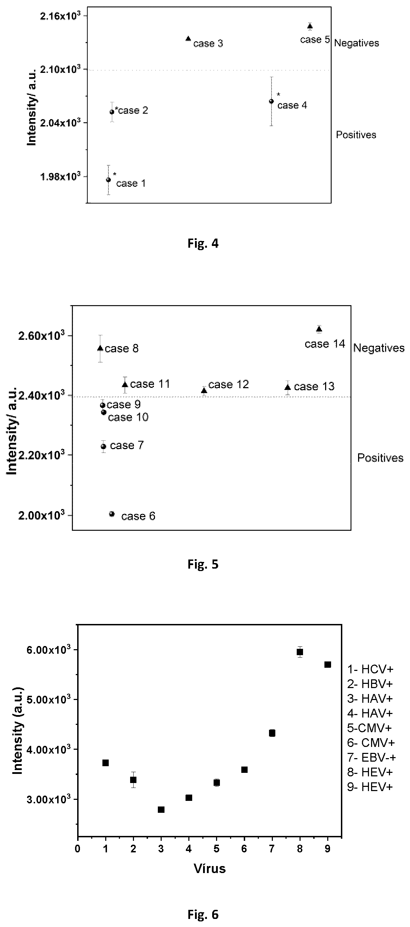

Figure 4 shows the specificity results obtained for HEV detection in human serum samples. These results were in 100% agreement with RT-qPCR.

Figure 5 discloses results obtained for 4 HEV positive faeces samples and 5 negatives using the described procedure and the System of the present disclosure. These results are in 100% agreement with RT-qPCR.

**Figure 6** shows selectivity assays of the diagnostic kits using human serum samples, where the samples identified from 1-17 are: HCV+, HBV+, HAV+ 1, HAV+_2, CMV+_1, CMV+_2, EBV+, HEV+_1, HEV+_2, respectively, using the JK probe diagnostic kit. Each point corresponds to the mean obtained from at least 3 measurements. *Values are statistically significant at a 5% confident level.

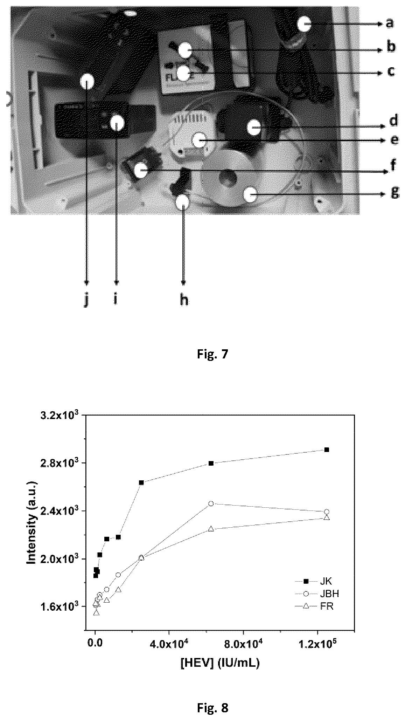

**Figure 7** is an Internal view of the hardware used on the System of the present disclosure unit. Where: **a**- USB cable that connects the spectrometer unit to the mini computer or external portable computer (depending on the final configuration of the biosensor); **b**- optical fiber (quartz core); **c**-spectrometer unit; **d**-sampler unit where cuvettes are inserted for measurement; **e**-transistor for LED; **f**-On/OFF switch button; **g**- Heating unit with controlling probe that is connected to the j unit for adjusting the heating conditions. Depending on the biosensor final configuration this heating unit has a pressure sensor on the top, connected to the software controlling the cadency of the steps to be performed in order to obtain a valid measurement. It serves the point of informing the software that the time required for heating can start; **h**- LED; **i**-Timmer unit (depending on the final configuration of the biosensor, this unit may not be necessary as it is incorporated in the software; **j**- Unit that controls the temperature probe inside the heating unit, hence controlling the temperature.

**Figure 8** is Polynomial fit of the S curve for the HEV diagnostic kit using WHO standard dilutions.

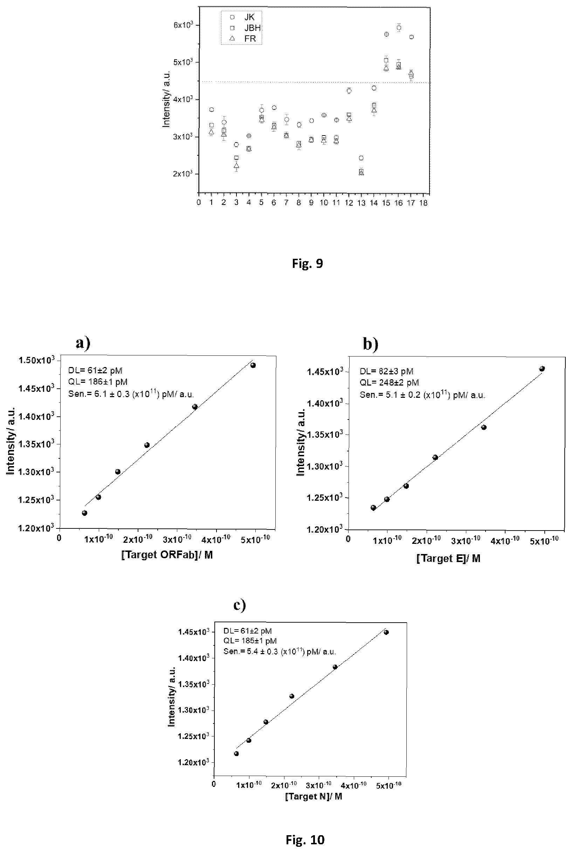

**Figure 9** shows selectivity assays of the diagnostic kits using human serum samples, where the samples identified from 1-17 are: HCV+, HBV+, HAV+_1, HAV+_2, HAV+_3, HAV+_4, HAV+_5, CMV+_1, CMV+_2, CMV+_3, CMV+_4, CMV+_5, CMV+_6, EBV+, HEV+_1, HEV+_2, HEV+_3, respectively. Each point corresponds to the mean obtained from at least 3 measurements. * Values are statistically significant at a 5% confident level.

**Figure 10** is a regression analysis for the probes a) Orf1ab, b) E and c) N, with the inset demonstrating the limit of detection (DL), quantification (QL) and method sensitivity (Sen.).

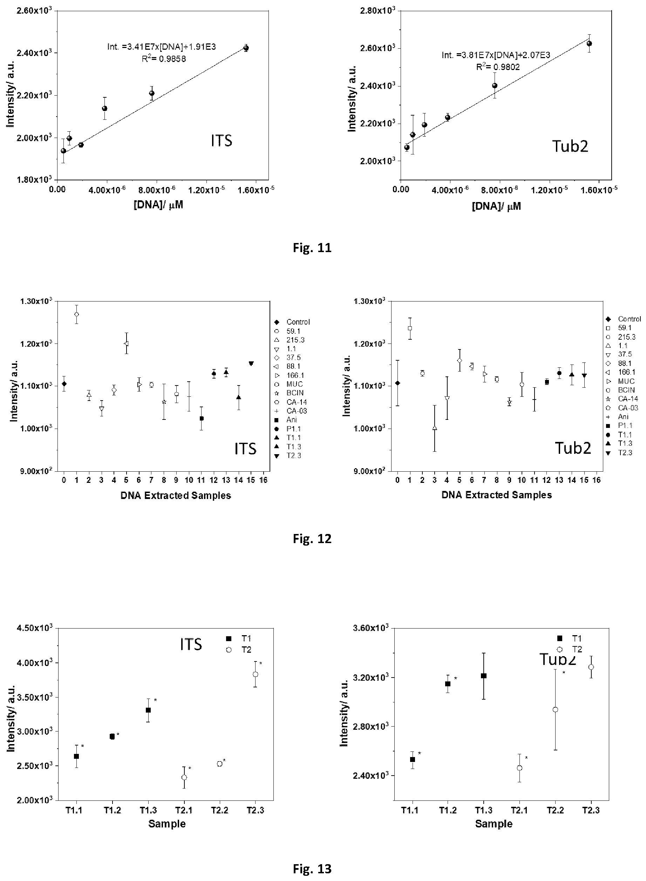

**Figure 11** is a regression analysis for the probes using DNA sample of Eutypa lata isolate a) ITS probe, b) Tub2 probe. Each point corresponds to at least three independent measurements.

**Figure 12** is a specificity assay using the System of the present disclosure in several swabs of fungal isolates *E. lata* (59.1), *N. parvum* (215.3), *D. seriata* (1.1), *N. luteum* (37.5), D. ampelina (88.1), *F. mediterranea* (166.1), *Mucor spp.* (MUC), *B. cinerea* (BCIN), A. alternata (CA-14), *C. fiorinae* (CA-03), A. niger (Ani), *Penicilliums spp.* (P1.1), P. minimum (T1.1), *F. mediterranea* (T1.3), and *P. chlamydospore* (T2.3) with the; a) ITS probe; b) Tub2 probe. Each point corresponds to the mean obtained from at least 3 measurements.

**Figure 13** shows the results of the analysis of the System of the present disclosure platform testing sawdust samples harvested from cv. Arinto vines from trunk 1 (T1.1, T1.2, T1.3), and from trunk 2 (T2.1, T2.2, T2.3) with a) ITS probe; b) Tub2 probe. Each point corresponds to the mean obtained from 3 measurements. *Values are statistically significant at a 5% confidence level.

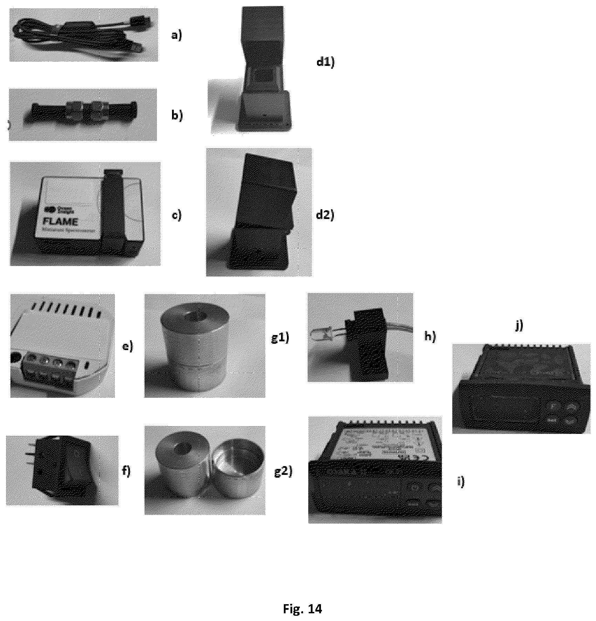

**Figure 14** shows the several options of individual components which are suitable to be integrated in the device of the present invention, namely: a- USB cable that connects the spectrometer unit to the mini computer or external portable computer (depending on the final configuration of the biosensor); b-optical fiber (quartz core); c- spectrometer unit; d- sampler unit where cuvettes are inserted for measurement; e-transistor for LED; f- On/OFF switch button; g- Heating unit with controlling probe that is connected to the j unit for adjusting the heating conditions. Depending on the biosensor final configuration this heating unit has a pressure sensor on the top, connected to the software controlling the cadency of the steps to be performed in order to obtain a valid measurement. It serves the point of informing the software that the time required for heating can start; h- LED; i-Timmer unit (depending on the final configuration of the biosensor, this unit may not be necessary as it is incorporated in the software; j- Unit that controls the temperature probe inside the heating unit, hence controlling the temperature.

## DETAILED DESCRIPTION

The more general and advantageous configurations of the present invention are described in the Summary of the invention. Such configurations are detailed below in accordance with other advantageous and/or preferred embodiments of implementation of the present invention.

In an embodiment, it is necessary to insert 5 μL of an inactivation solution on a calibration kit of a given diagnostic kit. The calibration kit comprises a tube with an inactivation solution, three calibration solutions and three micropipettes to extract the 5 μL. It is necessary to allow for 5 min incubation and then insert the calibration kit on the system. Depending if the system has one or three chambers it will be necessary to insert one at the time or all at once. If the system has only one chamber, the software will light up the code for the insertion of calibration solutions: A, then B and C. If the system has three chambers, the solutions need to be inserted by the same order (from left to right - A, B, C).

In a further embodiment, the system of the present disclosure comprises a box, a display; a mini-integrated computer with internal and external memory and wireless plate; a heating unit; a sample holder; an optical unit that is responsible for receiving the excitation wavelength, from the sampler, through an adequate optical fiber, and transmit the emission wavelength to the computer (in an embodiment, this connection is performed by cable or optical fiber), a light emitter, which is LED or other excitation source, according to the intended application.

In a further embodiment, the diagnostic kit comprises a tube with the inactivation solution (the biological sample should be collected and inserted in this tube), three testing solutions and 3 micropipettes. The biological sample is provided in a tube along with the inactivation solution (to assure the biological safety of the sample, which allows the application of the analysis *in situ*). This tube with the biological sample is inserted into the heating chamber for a given amount of time (according to the manufacturer instructions for each specific kit). Afterwards, a similar procedure to calibration is performed, where 5 μL of the heated solution is inserted on the three testing solutions and left to incubate for 5 min. Then the solutions are inserted into the chamber/s using the same procedure of the calibration. Afterwards, the software analyses the signal and processes the information to provide a result: positive, negative or inconclusive. When an inconclusive result is obtained, the user is requested to start the procedure from the beginning, including the sample collection.

In an embodiment, several components are integrated in the present device, such as any suitable data integration system. A computer, a tablet or an external disk are examples of such components. This feature is very relevant as it turns the present disclosure very flexible and easily adaptable to any source of data management suitable to be integrated in the present device.

In a further embodiment several components are integrated in the present device, such as any suitable component for centrifugation, particularly suitable to centrifuge samples at a maximum speed from 6000 rpm to 12000 rpm, which is incorporated inside the System of the present disclosure box or used externally, depending on the final System of the present disclosure layout.

In order to evaluate the analytical parameters of the diagnostic kit a series of sequential dilutions were performed, using a WHO standard (Figure 8). From its analysis it was possible to calculate the detection and quantification limits, along with the sensitivity of this method, i.e., the ability that the method has to discriminate between close samples (Table 2).

Table 2. Analytical parameters for the HEV (Hepatitis E Virus) diagnostic kits

| | LOD (IU. mL$^{-1}$.a.u$^{-1}$) | LOQ (IU. mL$^{-1}$.a.u$^{-1}$) | Sensitivity (IU$^{-1}$.mL·a.u) |
|---|---|---|---|
| **JK_Kit** | 0.4±0.2 | 1.3±0.1 | 0.049±0.006 |
| **JBH_Kit** | 0.6±0.2 | 2.0±0.1 | 0.022±0.004 |
| **FR_Kit** | 0.8±0.3 | 2.5±0.2 | 0.027±0.007 |

To evaluate the selectivity of the diagnostic kits, human serum samples were used (Figure 9). These samples were previously tested for HEV using RT-PCR. For this assay we used the same lot of samples. As all previous tests, the selectivity assays were performed by two researchers and the results validated by both at the time of measurement.

The data obtained (Figure 5) prove that using the system of the present disclosure allows the achievement of a positive or negative result which are in agreement with the one obtained using RT-PCR that is the state of the art most common method for this diagnosis. As such, the efficacy of the system of the present disclosure is highlighted.

**EXAMPLES**

**Example 1: HEV (Hepatitis E Virus) detection in serum samples**

Initially 10 μL of the individual serum samples were combined with 90 μL of diluent, which could consist of PBS, PCR-grade water, distilled water, lysis buffer, 70% ethanol, or RNA Stand-By Solution. This sample mixture was prepared in duplicate and processed using fresh reagents.

Afterwards it was heated at 95°C for 1 min using the System of the present disclosure units g), i) and j) (Figure 1). The diagnostic kit was composed by three solutions. Each solution had a specific probe for HEV detection. These probes were previously described for this application using RT-qPCR (Jothikumar et al. 2006; Frías et al. 2021; Hinrichs et al. 2024) (Table 1). The sample mixture was added to each kit solution and left to incubate for 5 min at room temperature. Upon incubation the kit was placed on a disposable cuvette and measured on the System of the present disclosure fluorescent equipment (unit d) Figure 1). The LED excites (unit h) Figure 1) the nanoparticles on the diagnostic kit, the optical signal is sent to the spectrometer through the optical fiber (unit b) Figure 1) and the spectrometer (unit c) Figure 1) converts this signal into a measurable output. This signal is sent into the computer (unit i) or ii) Figure 2) and converted into fluorescence variation using the spectrometer manufacturer software. This signal is then viewed by the user on the tablet or portable computer (unit ii) Figure 2).

**Table 1.** Oligonucleotide sequences of probes used for HEV detection.

| Target region of HEV | Oligonucleotide | Sequence (5'-3') |
|---|---|---|
| ORF3 | JVHEVP | TGATTCTCAGCCCTTCGC |
| ORF3 | FHEVP | TGAYTCYCARCCCTTCGC |
| ORF1 | JBHHEVP | CTGCTCTGGCTGCGGCCAA |

## Example 2: HEV detection in faeces samples

Faeces are a complex biological matrix that requires an extra pre-step when compared to the previous described HEV application (Example 1).

Initially, ultrapure water was added to faeces samples (the minimum possible - 10 μL to 1 mL) and the resulting solution was centrifuged (12000-14000 rpm) for 1 min (unit that can be inside the System of the present disclosure box, or used externally). The obtained clear solution was heated (95ºC) for 1 min (units g), i) and j), Figure 7) and then added to the testing kit. The sample mixture and kit solution were left to incubate for 5 min at room temperature. Upon incubation the kit was placed on a disposable cuvette and measured on the System of the present disclosure fluorescent equipment (unit d) Figure 7). The LED excites (unit h) Figure 7) the nanoparticles on the diagnostic kit, the optical signal is sent to the spectrometer through the optical fiber (unit b) Figure 7) and the spectrometer (unit c) Figure 7) converts this signal into a measurable output. This signal is sent into the computer (unit i) or ii) Figure 8) and converted into fluorescence variation using the spectrometer manufacturer software. This signal is then viewed by the user on the tablet or portable computer (unit ii) Figure 8).

The samples used in Example 1 and 2 are real biological samples that were previously tested by RT-qPCR. The result was unknown to the person performing the test using the system of the present disclosure's equipment. Positive and Negative samples were previously selected and given for testing.

## Example 3: SARS-CoV-2 detection

In this example naso/oropharyngeal swabs were used in biological sample preservative. The test begins by using heating the vial with the naso/oropharyngeal swab+preservative at 95ºC for 1 min (units g), i) and j), Figure 1). Afterwards, 5 μL of the swab solution was added to the testing kit. This testing kit, as described for Examples 1 and 2, were the ones already described for RT-qPCR for SARS-CoV-2 detection (Table 3). The sample mixture and kit solution were left to incubate for 5 min at room temperature. Upon incubation the kit was placed on a disposable cuvette and measured on the System of the present disclosure fluorescent equipment (unit d) Figure 1). The LED excites (unit h) Figure 1) the nanoparticles on the diagnostic kit, the optical signal is sent to the spectrometer through the optical fiber (unit b) Figure 1) and the spectrometer (unit c) Figure 1) converts this signal into a measurable output. This signal is sent into the computer (unit i) or ii) Figure 2) and converted into fluorescence variation using the spectrometer manufacturer software. This signal is then viewed by the user on the tablet or portable computer (unit ii) Figure 2).

**Table 4:** Oligonucleotides used as probes for the development of the diagnostic kit and RT-qPCR results for SARS-CoV-2

| Oligonucleotide | Sequence (5'-3') | Reference |
|---|---|---|
| ORF1ab_P_DNA | CCG TCT GCG GTA TGT GGA AAG GTT ATG G | (Victor M Corman et al. 2020) |
| E_Sarbeco_P_DNA | ACA CTA GCC ATC CTT ACT GCG CTT CG | |
| N_P_DNA | TTG CTG CTG CTT GAC AGA TT | |

The analytical parameters of the diagnostic kit using the System of the present disclosure were determined and the data are showed in Figure 10.

Other analytical studies were performed in order to validate the overall efficiency of this methodology, namely a linearity study, a lot-to-lot study and an assay precision study.

This study objective was to show the dilution linearity of the developed SARS-CoV-2 detection kit. To do so, one lot of samples was randomly selected and used.

Three positive samples diluted in water, generating at least six, 2-fold, consecutive dilutions were prepared for each sample. The assays were performed according to the principles and guidelines of good manufacturing practice (GMP) for medicinal products. The study was designed in alignment with the CLSI I/LA20-Ed3, Appendix A. Initially, to establish a standard deviation, each undiluted sample was independently measured twelve times, then each dilution was replicated 4 times and measured independently.

Calculations

The intra assay %CV between replicates for each sample dilution was calculated as:

$$\%CV = 100 \times (\text{standard deviation})/(\text{mean concentration}).$$

The intra assay %CV (over all dilutions) and inter dilutional %CV for each sample was calculated using Analysis of Variance (REML method).

Regression analysis was calculated according to:

$$\text{Ln (observed concentration)} = a + b \cdot \text{Ln (expected concentration)}$$

where: a = intercept, b = slope

The expected concentration (kUA/L) was calculated as undiluted mean divided by the dilution factor. The recovery (%) for each sample and dilution was calculated as the observed concentration (O) divided by the expected concentration (E) according to: O/E x 100.

Specifications

Intra assay CV shall be < 12% for sample concentrations $\geq$ 0.1 kUA/L

Inter dilutional CV shall be < 20%

Observed/Expected should be between 0.8 and 1.2 for all samples

Regression analysis slope should be 1.0 $\pm$ 0.1

Regression analysis intercept should be 0.0 $\pm$ 0.23

Table 5. SARS-CoV-2 detection kit, Intra assay and inter dilutional CV values

| Positive1 | CV/ % Interdilutional | | |
|---|---|---|---|
| Concentration/ kUA.L$^{-1}$ | Orf | E | N |
| 40.00 | 2.4 | 0.7 | 1.3 |
| 28.00 | 0.4 | 0.7 | 1.3 |
| 18.00 | 0.5 | 0.9 | 0.8 |
| 12.00 | 0.6 | 0.9 | 0.2 |
| 8.00 | 0.9 | 0.5 | 0.8 |
| 1.56 | 0.8 | 0.5 | 0.3 |
| 1.00 | 0.8 | 1.0 | 0.5 |
| Positive2 | CV/ % Interdilutional | | |
| Concentration/ kUA.L$^{-1}$ | Orf | E | N |
| 40.00 | 1.3 | 1.5 | 1.5 |
| 28.00 | 1.3 | 0.5 | 0.7 |

(continued)

| Positive2 | CV/ % Interdilutional | | |
|---|---|---|---|
| Concentration/ kUA.L$^{-1}$ | Orf | E | N |
| 18.00 | 1.0 | 1.0 | 0.1 |
| 12.00 | 0.3 | 1.6 | 0.3 |
| 8.00 | 0.7 | 0.7 | 0.8 |
| 1.56 | 1.2 | 0.4 | 0.3 |
| 1.00 | 0.8 | 1.4 | 1.2 |
| Positive3 | CV/ % Interdilutional | | |
| Concentration/ kUA.L$^{-1}$ | Orf | E | N |
| 40.00 | 1.8 | 1.5 | 4.6 |
| 28.00 | 0.8 | 0.6 | 0.7 |
| 18.00 | 0.8 | 0.7 | 0.7 |
| 12.00 | 0.5 | 0.6 | 0.6 |
| 8.00 | 0.7 | 0.5 | 0.7 |
| 1.56 | 0.3 | 1.2 | 0.5 |
| 1.00 | 0.9 | 1.5 | 1.1 |
| | CV/ % Intra dilutional | | |
| Concentration/ kUA.L$^{-1}$ | Orf | E | N |
| 40.00 | 4.4 | 3.8 | 3.1 |
| 28.00 | 4.1 | 4.4 | 3.5 |
| 18.00 | 3.1 | 3.1 | 1.8 |
| 12.00 | 1.7 | 2.3 | 1.5 |
| 8.00 | 1.5 | 1.1 | 2.4 |
| 1.56 | 1.7 | 1.5 | 1.4 |
| 1.00 | 1.0 | 1.5 | 2.4 |

**Table 6.** Regression analysis

| Sample | Target | Intercept | SD | Slope | SD | R$^2$ |
|---|---|---|---|---|---|---|
| Positive1 | ORF | 0.0 | 0.274 | 1.0 | 0.013 | 0.9992 |
| | E | -0.6 | 0.350 | 1.1 | 0.017 | 0.9988 |
| | N | -0.9 | 1.05 | 1.1 | 0.052 | 0.9895 |
| Positive2 | ORF | 0.2 | 0.352 | 1.0 | 0.017 | 0.9987 |
| | E | -1.0 | 1.29 | 1.1 | 0.064 | 0.9844 |
| | N | -0.8 | 1.02 | 1.1 | 0.050 | 0.9899 |
| Positive3 | ORF | 0.7 | 0.612 | 1.0 | 0.029 | 0.9960 |
| | E | 0.6 | 0.688 | 1.0 | 0.032 | 0.9949 |
| | N | 0.5 | 0.639 | 1.0 | 0.030 | 0.9957 |

**Table 7.** Summary for samples and dilution

| Sample | Target | Expected Concentration (kUA.L-1) | Observed Concentration (kUA.L-1) | % R (O/E) |
|---|---|---|---|---|
| Positive1 | ORF | 38.49 | 40.00 | 1.0 |

(continued)

| Sample | Target | Expected Concentration (kUA.L-1) | Observed Concentration (kUA.L-1) | % R (O/E) |
|---|---|---|---|---|
| | E | 36.56 | 40.00 | 1.1 |
| | N | 34.67 | 40.00 | 1.2 |
| | ORF | 26.24 | 28.00 | 1.1 |
| | E | 26.20 | 28.00 | 1.1 |
| | N | 27.08 | 28.00 | 1.0 |
| | ORF | 17.36 | 18.00 | 1.0 |
| | E | 17.50 | 18.00 | 1.0 |
| | N | 17.92 | 18.00 | 1.0 |
| | ORF | 11.84 | 12.00 | 1.0 |
| | E | 11.77 | 12.00 | 1.0 |
| | N | 11.13 | 12.00 | 1.1 |
| | ORF | 7.42 | 8.00 | 1.1 |
| | E | 7.69 | 8.00 | 1.0 |
| | N | 7.78 | 8.00 | 1.0 |
| | ORF | 1.55 | 1.56 | 1.0 |
| | E | 1.55 | 1.56 | 1.0 |
| | N | 1.53 | 1.56 | 1.0 |
| Positive2 | ORF | 38.52 | 40.00 | 1.0 |
| | E | 33.80 | 40.00 | 1.2 |
| | N | 35.00 | 40.00 | 1.1 |
| | ORF | 27.81 | 28.00 | 1.0 |
| | E | 27.31 | 28.00 | 1.0 |
| | N | 27.29 | 28.00 | 1.0 |
| | ORF | 16.74 | 18.00 | 1.1 |
| | E | 17.54 | 18.00 | 1.0 |
| | N | 17.98 | 18.00 | 1.0 |
| | ORF | 11.52 | 12.00 | 1.0 |
| | E | 11.46 | 12.00 | 1.0 |
| | N | 11.35 | 12.00 | 1.1 |
| | ORF | 7.64 | 8.00 | 1.0 |
| | E | 7.74 | 8.00 | 1.0 |
| | N | 7.63 | 8.00 | 1.0 |
| | ORF | 1.50 | 1.56 | 1.0 |
| | E | 1.49 | 1.56 | 1.1 |
| | N | 1.45 | 1.56 | 1.1 |
| Positive3 | ORF | 40.00 | 40.00 | 1.0 |
| | E | 40.00 | 40.00 | 1.0 |
| | N | 40.00 | 40.00 | 1.0 |
| | ORF | 26.88 | 28.00 | 1.0 |

(continued)

| Sample | Target | Expected Concentration (kUA.L-1) | Observed Concentration (kUA.L-1) | % R (O/E) |
|--------|--------|----------------------------------|----------------------------------|-----------|
|  | E | 25.77 | 28.00 | 1.1 |
|  | N | 25.79 | 28.00 | 1.1 |
|  | ORF | 15.90 | 18.00 | 1.1 |
|  | E | 16.43 | 18.00 | 1.1 |
|  | N | 16.83 | 18.00 | 1.1 |
|  | ORF | 11.31 | 12.00 | 1.1 |
|  | E | 11.35 | 12.00 | 1.1 |
|  | N | 11.53 | 12.00 | 1.0 |
|  | ORF | 7.70 | 8.00 | 1.0 |
|  | E | 7.72 | 8.00 | 1.0 |
|  | N | 7.68 | 8.00 | 1.0 |
|  | ORF | 1.55 | 1.56 | 1.0 |
|  | E | 1.53 | 1.56 | 1.0 |
|  | N | 1.49 | 1.56 | 1.1 |

This study was designed to evaluate the lot-to-lot reproducibility of the SARS-CoV-2 detection kit. To do so, three independent lots were randomly selected and 3 positive samples ($0.35 \pm 25\%$ kUA/L, $\geq 0.7$ kUA/L and 10-20 kUA/L) and 1 negative sample (< 0.1 kUA/L) were tested. For each lot the samples were tested in 12 replicates in one assay run.

The assays were performed according to the principles and guidelines of good manufacturing practice (GMP) for medicinal products.

Calculation

Mean concentration values, %CV within run and concentration quotients between lots were calculated for the positive samples. Coefficients of variation (%CV) for the concentrations were calculated as: %CV = (standard deviation)/(mean concentration)*100

Specifications

Positive samples: Quotients in concentration (lotmax/lotmin) shall be <1.3; Within run CV shall be < 12% for sample concentrations > 0.1 kUA/L; Negative sample: All results shall be < 0.1 kUA/L.

The results for the samples fulfilled the specifications for all three components. The data collected through the experiences and their interpretation can be found below.

**Table 8.** Mean and CV for each positive and negative sample for each of the three lots used.

| Lot1 | Mean Positive1 (15 kUA/L) /Int. - CV% | Mean Positive2 (0.60 kUA/L)/ Int. - CV% | Mean Positive3 (0.3 kUA/L)/ Int. - CV% | Mean Negative (0.11 kUA/L)/ Int. - CV% |
|------|---------------------------------------|-----------------------------------------|----------------------------------------|----------------------------------------|
| ORF | 1612 - 1.8 | 1428 - 1.4 | 1384 - 1.5 | 1241 - 1.9 |
| E | 1618 - 1.6 | 1593 - 2.2 | 1542 - 1.8 | 1185 -1.6 |
| N | 1538 -1.5 | 1505 - 1.7 | 1403 - 1.4 | 1170-2.5 |
| Lot2 | Mean Positive1 (15 kUA/L) /Int. - CV% | Mean Positive2 (0.60 kUA/L)/ Int. - CV% | Mean Positive3 (0.3 kUA/L)/ Int. - CV% | Mean Negative (0.11 kUA/L)/ Int. - CV% |
| ORF | 1621 - 2.8 | 1595 - 4.1 | 1418 - 1.6 | 1150 - 1.3 |
| E | 1545 - 1.3 | 1471-1.2 | 1450 - 1.4 | 1031-1.5 |
| N | 1517 -1.2 | 1471-1.3 | 1422 - 1.3 | 1128 -1.1 |

(continued)

| Lot3 | Mean Positive1 (15 kUA/L) /Int. - CV% | Mean Positive2 (0.60 kUA/L)/ Int. - CV% | Mean Positive3 (0.3 kUA/L)/ Int. - CV% | Mean Negative (0.11 kUA/L)/ Int. - CV% |
|---|---|---|---|---|
| ORF | 1592 - 1.6 | 1566 - 1.5 | 1463 - 1.2 | 1073 - 1.6 |
| E | 1715 - 2.1 | 1453 - 1.4 | 1428 -1.3 | 1208 -1.1 |
| N | 1626 - 1.9 | 1477 - 1.8 | 1451-1.3 | 1162 - 1.5 |

**Table 9.** Values of min, max and quotient for the positive samples considering the three lots used.

| | Lot 1 | | |
|---|---|---|---|
| ORF | Min/ Int. | Max/ Int. | Max/Min |
| Positive 1 (15 kUA/L) | 1561 | 1656 | 1.1 |
| Positive 2 (0.60 kUA/L) | 1399 | 1461 | 1.0 |
| Positive 3 (0.3 kUA/L)/ | 1345 | 1413 | 1.1 |
| | Lot 2 | | |
| ORF | Min/ Int. | Max/ Int. | Max/Min |
| Positive 1 (15 kUA/L) | 1536 | 1670 | 1.1 |
| Positive 2 (0.60 kUA/L) | 1502 | 1690 | 1.1 |
| Positive 3 (0.3 kUA/L)/ | 1379 | 1456 | 1.1 |
| | Lot 3 | | |
| ORF | Min/ Int. | Max/ Int. | Max/Min |
| Positive 1 (15 kUA/L) | 1550 | 1630 | 1.1 |
| Positive 2 (0.60 kUA/L) | 1537 | 1600 | 1.0 |
| Positive 3 (0.3 kUA/L)/ | 1442 | 1507 | 1.0 |
| | Lot 1 | | |
| E | Min/ Int. | Max/ Int. | Max/Min |
| Positive 1 (15 kUA/L) | 1573 | 1658 | 1.1 |
| Positive 2 (0.60 kUA/L) | 1546 | 1661 | 1.1 |
| Positive 3 (0.3 kUA/L)/ | 1501 | 1586 | 1.1 |
| | Lot 2 | | |
| E | Min/ Int. | Max/ Int. | Max/Min |
| Positive 1 (15 kUA/L) | 1512 | 1583 | 1.0 |
| Positive 2 (0.60 kUA/L) | 1435 | 1497 | 1.0 |
| Positive 3 (0.3 kUA/L)/ | 1418 | 1479 | 1.0 |
| | Lot 3 | | |
| E | Min/ Int. | Max/ Int. | Max/Min |
| Positive 1 (15 kUA/L) | 1651 | 1788 | 1.1 |
| Positive 2 (0.60 kUA/L) | 1426 | 1496 | 1.0 |
| Positive 3 (0.3 kUA/L)/ | 1387 | 1454 | 1.0 |
| | Lot 1 | | |
| N | Min/ Int. | Max/ Int. | Max/Min |
| Positive 1 (15 kUA/L) | 1502 | 1565 | 1.0 |

(continued)

| N | Lot 1 | | |
|---|---|---|---|
| | Min/ Int. | Max/ Int. | Max/Min |
| Positive 2 (0.60 kUA/L) | 1472 | 1552 | 1.1 |
| Positive 3 (0.3 kUA/L)/ | 1371 | 1429 | 1.0 |
| N | Lot 2 | | |
| | Min/ Int. | Max/ Int. | Max/Min |
| Positive 1 (15 kUA/L) | 1482 | 1543 | 1.0 |
| Positive 2 (0.60 kUA/L) | 1435 | 1497 | 1.0 |
| Positive 3 (0.3 kUA/L)/ | 1379 | 1456 | 1.0 |
| N | Lot 3 | | |
| | Min/ Int. | Max/ Int. | Max/Min |
| Positive 1 (15 kUA/L) | 1568 | 1678 | 1.1 |
| Positive 2 (0.60 kUA/L) | 1435 | 1520 | 1.1 |
| Positive 3 (0.3 kUA/L)/ | 1420 | 1482 | 1.0 |

The System of the present disclosure results were also compared to the ones obtained by RT-qPCR, in order to validate its efficiency when compared to the golden standard technique for viral diagnostic. The results are presented in Table 10-12.

Table 10: Comparison between the positive results obtained using the System of the present disclosure and RT-qPCR for extracted RNA from real samples using the same target regions, ORF1ab, E and N.

| | System of the present disclosure Results | | | | RT-qPCR Result (Ct) | | | |
|---|---|---|---|---|---|---|---|---|
| Sample | ORFlab | E | N | Overall Result | ORFlab | E | N | Overall Result |
| 1 | + | - | + | Positive | 29 | 29 | 26 | Positive |
| 2 | + | - | + | Positive | 29 | 29 | 26 | Positive |
| 3 | + | - | + | Positive | - | 35 | 34 | Positive |
| 4 | + | + | + | Positive | 26 | 26 | 24 | Positive |
| 5 | + | + | - | Positive | 32 | 33 | 31 | Positive |
| 6 | + | + | + | Positive | 26 | 26 | 24 | Positive |
| 7 | + | + | + | Positive | 30 | 31 | 29 | Positive |
| 8 | + | + | + | Positive | 33 | 35 | 31 | Positive |
| 9 | + | + | + | Positive | 32 | 32 | 32 | Positive |
| 10 | + | + | + | Positive | 29 | 28 | 27 | Positive |
| 11 | + | + | + | Positive | 33 | 33 | 30 | Positive |
| 12 | + | + | + | Positive | 33 | 33 | 29 | Positive |
| 13 | + | + | + | Positive | 26 | 25 | 23 | Positive |
| 14 | + | - | + | Positive | 30 | 31 | 29 | Positive |
| 15 | + | + | + | Positive | 31 | 31 | 29 | Positive |
| 16 | + | + | + | Positive | 35 | 36 | 14 | Positive |
| 17 | + | - | + | Positive | 33 | 35 | 32 | Positive |
| 18 | + | + | - | Positive | 33 | 34 | 31 | Positive |
| 19 | + | + | + | Positive | 33 | 33 | 30 | Positive |

(continued)

| | System of the present disclosure Results | | | | RT-qPCR Result (Ct) | | | |
|---|---|---|---|---|---|---|---|---|
| Sample | ORFlab | E | N | Overall Result | ORFlab | E | N | Overall Result |
| 20 | + | + | - | Positive | 29 | 28 | 26 | Positive |
| 21 | + | + | - | Positive | 10 | 38 | 33 | Positive |
| 22 | + | - | + | Positive | 34 | 34 | 31 | Positive |
| 23 | - | + | + | Positive | - | 35 | 34 | Positive |
| 24 | + | + | - | Positive | 36 | 33 | 31 | Positive |
| 25 | + | + | + | Positive | 27 | 28 | 26 | Positive |
| 26 | + | + | + | Positive | 30 | 31 | 29 | Positive |
| 27 | + | + | + | Positive | 35 | 36 | 33 | Positive |
| 28 | - | + | + | Positive | 26 | 27 | 24 | Positive |
| 29 | + | + | + | Positive | 29 | 29 | 27 | Positive |
| 30 | - | + | + | Positive | 28 | 28 | 26 | Positive |
| 31 | + | + | + | Positive | 32 | 31 | 29 | Positive |
| 32 | + | + | - | Positive | 31 | 31 | 29 | Positive |
| 33 | + | + | + | Positive | 36 | 10 | 35 | Positive |
| 34 | + | + | - | Positive | 26 | 25 | 22 | Positive |
| 35 | + | + | + | Positive | 36 | 34 | 32 | Positive |
| 36 | + | - | + | Positive | 34 | 37 | 34 | Positive |
| 37 | + | + | - | Positive | 32 | 32 | 30 | Positive |

**Table 11:** Comparison between the negative results obtained using the System of the present disclosure and RT-qPCR for extracted RNA from real samples using the same target regions, ORF1ab, E and N.

| | System of the present disclosure Results | | | | RT-qPCR Result (Ct) | | | |
|---|---|---|---|---|---|---|---|---|
| Sample | ORFlab | E | N | Overall Result | ORFlab | E | N | Overall Result |
| 1 | - | + | - | Negative | - | - | - | Negative |
| 2 | - | - | - | Negative | - | - | - | Negative |
| 3 | - | - | + | Negative | - | - | - | Negative |
| 4 | - | - | - | Negative | - | - | - | Negative |
| 5 | - | - | - | Negative | - | - | - | Negative |
| 6 | - | - | - | Negative | - | - | - | Negative |
| 7 | - | - | - | Negative | - | - | - | Negative |
| 8 | - | - | - | Negative | - | - | - | Negative |
| 9 | + | - | - | Negative | - | - | - | Negative |
| 10 | - | - | - | Negative | - | - | - | Negative |
| 11 | - | - | + | Negative | - | - | - | Negative |
| 12 | + | - | - | Negative | - | - | - | Negative |
| 13 | + | - | - | Negative | - | - | - | Negative |
| 14 | - | - | - | Negative | - | - | - | Negative |
| 15 | - | - | + | Negative | - | - | - | Negative |
| 16 | + | - | - | Negative | - | - | - | Negative |

(continued)

| | System of the present disclosure Results | | | | RT-qPCR Result (Ct) | | | |
|---|---|---|---|---|---|---|---|---|
| Sample | ORFlab | E | N | Overall Result | ORFlab | E | N | Overall Result |
| 17 | - | - | - | Negative | - | - | - | Negative |
| 18 | - | + | - | Negative | - | - | - | Negative |
| 19 | + | - | - | Negative | - | - | - | Negative |
| 20 | - | + | - | Negative | - | - | - | Negative |
| 21 | - | + | - | Negative | - | - | - | Negative |
| 22 | + | - | - | Negative | - | - | - | Negative |
| 23 | + | - | - | Negative | - | - | - | Negative |
| 24 | - | - | + | Negative | - | - | - | Negative |
| 25 | - | - | + | Negative | - | - | - | Negative |
| 26 | + | - | - | Negative | - | - | - | Negative |
| 27 | - | - | - | Negative | - | - | - | Negative |
| 28 | - | - | + | Negative | - | - | - | Negative |
| 29 | - | - | + | Negative | - | - | - | Negative |
| 30 | - | - | - | Negative | - | - | - | Negative |
| 31 | - | - | - | Negative | - | - | - | Negative |
| 32 | - | - | - | Negative | - | - | - | Negative |
| 33 | - | - | - | Negative | - | - | - | Negative |
| 34 | - | - | - | Negative | - | - | - | Negative |
| 35 | + | - | - | Negative | - | - | - | Negative |
| 36 | - | - | - | Negative | - | - | - | Negative |
| 37 | - | - | - | Negative | - | - | - | Negative |
| 38 | - | - | - | Negative | - | - | - | Negative |

**Table 12:** Comparison between the inconclusive results obtained using the System of the present disclosure and RT-qPCR for extracted RNA from real samples using the same target regions, ORF1ab, E and N.

| | System of the present disclosure Results | | | | RT-qPCR Result (Ct) | | | |
|---|---|---|---|---|---|---|---|---|
| Sample | ORFlab | E | N | Overall Result | ORFlab | E | N | Overall Result |
| 1 | - | + | - | Negative | 40 | 36 | 32 | Inconclusive |
| 2 | + | + | - | Positive | - | - | 38 | Inconclusive |
| 3 | + | + | + | Positive | 40.9 | 36 | 32.8 | Inconclusive |
| 4 | + | + | - | Positive | - | - | 38.8 | Inconclusive |
| 5 | + | - | + | Positive | - | - | 37.7 | Inconclusive |
| 6 | + | - | - | Negative | - | 37 | 35 | Inconclusive |
| 7 | - | - | - | Negative | - | 36.4 | 35 | Inconclusive |
| 8 | - | + | + | Positive | - | - | 36 | Inconclusive |
| 9 | + | + | - | Positive | - | 37 | 34 | Inconclusive |
| 10 | /* | - | + | Inconclusive | 36 | - | 36 | Inconclusive |
| 11 | + | - | - | Negative | 36 | 35 | 37 | Inconclusive |
| 12 | + | + | - | Positive | - | 38 | 35 | Inconclusive |

(continued)

| | System of the present disclosure Results | | | | RT-qPCR Result (Ct) | | | |
|---|---|---|---|---|---|---|---|---|
| Sample | ORFlab | E | N | Overall Result | ORFlab | E | N | Overall Result |
| 13 | + | + | + | Positive | - | 37 | 37 | Inconclusive |
| 14 | + | + | + | Positive | 38 | - | 34 | Inconclusive |
| 15 | - | + | + | Positive | - | 37 | 34 | Inconclusive |
| 16 | + | + | + | Positive | 38 | 44 | 33 | Inconclusive |
| *The obtained value was within the region of fluorescence natural fluctuations; hence no result was obtained for this sequence. | | | | | | | | |

This detection system composed by nanoparticles functionalized with genetic sequences to target the virus can be applied by non-specialized personnel using a portable system, that shows great promise for an advanced point-of-care diagnostic system. To assure high quality results, even in initial stages of infection, the genetic platform is composed by three vials, each one containing a genetic sequence complementary to a fragment of the virus. The results are interpreted as a whole. Two or more positive results indicate a positive sample and the inverse is also true for negative samples. The results obtained using SARS-CoV-2 naso/oropharyngeal swabs collected between May 2020 and March 2022, were compared in loco, with the data obtained by RT-qPCR. Initially, a 99.9% agreement was found between RT-qPCR results and the diagnostic kits developed; however, the only case in disagreement was later subjected to another naso/oropharyngeal swab collection and the initial positive result obtained using the kits was confirmed to be correct. Moreover, using this diagnostic kit throughout the testing period, no loss of sensitivity of the kit was verified, even though SARS-CoV-2 suffered many mutations during that time span.

**Example 4: Eutypa detection in *Vitis vinifera L* using extracted DNA, direct fungal swabs and sawdust**

Factors that influence grapevine value are related to adverse weather conditions and/or to the exposure to different pathogens. Also, fungal diseases have a great impact on the overall grape production and grape quality. Among the several fungal diseases, the ones included within the grapevine trunk diseases (GTDs) complex greatly contribute to a substantial loss in production. Eutypiose results from the infection of various fungi from different families, being the infection by *Eutypa lata* the major cause.

1) DNA samples extracted from fungal isolates were used. Here a 0.25 $\mu$M of the fungal was used.

2) Fungal material was also collected using a swab that was subsequently introduced into a 1.5 mL microtube containing 300 $\mu$L of water.

3) Sawdust samples were prepared by adding 300 $\mu$L of water to the microtube containing the sample.

The diagnostic kit was composed by solutions where the nanoparticles were functionalized with the oligonucleotides described in Table 13.

Initially, the sample was heated at 95°C for 1 min using the System of the present disclosure units g), i) and j) (Figure 1), immediately before use. This step is common to all biological matrixes used, i.e., extracted DNA, fungal swabs and sawdust. The sample was then added to each kit solution and left to incubate for 5 min at room temperature. Upon incubation the kit was placed on a disposable cuvette and measured on the System of the present disclosure fluorescent equipment (unit d) Figure 7). The **LED** excites (unit h) Figure 7) the nanoparticles on the diagnostic kit, the optical signal is sent to the spectrometer through the optical fiber (unit b) Figure 7) and the spectrometer (unit c) Figure 7) converts this signal into a measurable output. This signal is sent into the computer (unit i) or ii) Figure 8) and converted into fluorescence variation using the spectrometer manufacturer software. This signal is then viewed by the user on the tablet or portable computer (unit ii) Figure 8).

Table 13. Oligonucleotide sequences of probes used for *Eutypa lata* detection.

| Target region | Oligonucleotide | Sequence (5'-3') |
|---|---|---|
| ITS_279-308 | ITS | TGATTCTCAGCCCTTCGC |

(continued)

| Target region | Oligonucleotide | Sequence (5'-3') |
|---|---|---|
| Tub2_70-102 | Tub2 | TGAYTCYCARCCCTTCGC |

Degenerated nucleotides present in the sequence (Y), can be regarded as either Cytosine (C) or Thymine (T).

The analytical parameters of the diagnostic kit using DNA extracted samples in System of the present disclosure were evaluated (Figure 11).

Additionally, specificity assays for the different fungal species that are either involved in GTDs or appears in different types of materials as a secondary infection, were tested. Thus, an assay with swabs of several pure colonies of fungi were performed to establish a baseline that would allow a more accurate identification of Eutypa dieback pathogens with ITS and Tub2 targeting probes (Figure 12). In this experiment, a negative isolate was used as a control. Among all the samples, only sample 59.1 was positive for Eutypa lata. All samples were identified by the System of the present disclosure as negative. It is also interesting to notice that sample 88.1 produced a stronger fluorescence signal when compared to the other negative samples. This signal is attributed to the strong colouring of the sample that interferes with fluorescence reading, as it presents a black micelium. Nonetheless, this signal is still below 59.1 (E. lata) and therefore it is considered a negative sample.

In order to reduce the sample handling procedure, reducing the complexity of the assay, sawdust samples were directly tested in the System of the present disclosure platform. In the initial stage, sawdust obtained from 3 harvesting points on 2 different vine branches that were removed from infected cv. Arinto vines (Figure 13) were tested. The collection points were according from the health tissue (P1) to the infected tissues (P3). In both probes, there is a signal difference among the points; specifically, the fluorescence decreases from point 3 to 1.

The samples used in Example 3 and 4 are real biological samples were previously tested by RT-qPCR.

**Example 5** - Preparation of the System of the present disclosure for reading.

Heat the calibration sample for 1 min at 95ºC. Mix this sample with the calibration kit provided by the manufacturer and homogenize. Leave it to incubate for 5 min, then insert the calibration kit on the System of the present disclosure, using the genetic sequence cadence indicated by the diagnostic kit manufacturer.

A. Throughout the design of the diagnostic device of the present disclosure, more than 3000 samples were used to determine the confidence interval that defines a negative and a positive result. The previous protocol is applied equally for all. On step C, the sample is mixed, individually, with each of three given target genetic sequences. Hence the diagnostic is given through the overall analysis of the results provided for each target sequence.

B. Two positive sequences and one negative is consistent with a positive result and two negative sequences and one positive is consistent with a negative result.

The diagnostic is obtained through the comparison between the calibration solution and the sample, taking into consideration the confidence intervals defined by each kit. The results are calculated using a software that is integrated on the System of the present disclosure and does not require the user's manipulation. The result is showed as negative or positive. The individual results for each genetic sequence can also be observed.

**Example 6** - General procedure of the device of the present disclosure

A given diagnostic kit needs to be read by the device, via a QR code or de Code bar identification, in order for the software to start a protocol previously approved by the manufacturer.

When the software recognizes the kit, it is necessary to either: insert the patient data manually or read the code bar that was already attributed to that patient (upon sample collection or any pre-treatment, that is not performed by the system user) and which contains their relevant information.

A sample is then collected, for example inserting the swab into the mouth and rubbing it against the cheeks (oropharyngeal swab). Alternatively, a nasopharyngeal sample or a saliva sample can be collected. This sampling options allow the user to self-collect the sample with no need for the intervention of a health professional.

In an embodiment, other types of samples are envisaged, namely blood and stool samples.

Afterwards, the samples are placed in a vial with an inactivation solution. It is intended by inactivation solution a solution comprising chemical components that promote disruption of the viral/bacteria membranes/capsides and ensuring the virus/bacteria are no longer viable, such as, the already known BIOER PCR test.

The present disclosure was used with several probes summarized in Table 14, where "Y", "R" and "K" are degenerate nucleotides.

Table 14

| SEQ. ID No. | Probe | Sequence (5' to 3') | Diagnose |
|---|---|---|---|
| 1 | ORF1ab_P_DNA_s | CCG TCT GCG GTA TGT GGA AAG GTT ATG G | SARS-CoV-2 |
| 2 | E_Sarbeco_DNA_P1 | ACA CTA GCC ATC CTT ACT GCG CTT CG | SARS-CoV-2 |
| 3 | N_Gene_DNA_Probe | TTG CTG CTG CTT GAC AGA TT | SARS-CoV-2 |
| 4 | 2019-nCoV_N1-P | ACC CCG CAT TAC GTT TGG TGG ACC | SARS-CoV-2 |
| 5 | 2019-nCoV_N2-P | ACA ATT TGC CCC CAG CGC TTC AG | SARS-CoV-2 |
| 6 | 2019-nCoV_N3-P | AYC ACA TTG GCA CCC GCA ATC CTG | SARS-CoV-2 |
| 7 | HEV 1 | TGA TTC TCA GCC CTT CGC | HEV |
| 8 | HEV2 | RGR RGT TTC TGG GGT GAC | HEV |
| 9 | HEV3 | AKG GRT TGG TTG GRT GA | HEV |
| 10 | HEV 4 | TGA YTC YCA RCC CTT CGC | HEV |
| 11 | ITS | TGATTCTCAGCCCTTCGC | Grapvine Trunk Diseases |
| 12 | Tub2 | TGAYTCYCARCCCTTCGC | Grapvine Trunk Disease |
| 13 | ORF1 HEV | CTGCTCTGGCTGCGGCCAA | HEV |

In an embodiment, for calibrating the system of the present disclosure, it is necessary to insert 5 μL of an inactivation solution on the calibration kit solutions that are provided by the manufacturer along with the diagnostic kit. The calibration kit comprises: a tube with the inactivation solution, three calibration solutions and three micropipettes.

In an embodiment, the incubation step with the probes last for a maximum of 5 min, to assure probe-target hybridization, and then insert the calibration solutions kit on the system. Depending if the system has one or three chambers it will be necessary to insert one at the time or all at once. If the system has only one chamber, the software will light up the code for the insertion of calibration solution A, then B and C. If the system has three chambers, the solutions need to be inserted by the same order (from left to right - A, B, C).

In an embodiment, the samples' measurements are taken according to the following procedures:

1) Place the collecting vial with the swab into the heating chamber of the system for a given period of time that was established by the kits manufacturer (the device is equipped with a sensing pressure and the time starts counting automatically when the tube is correctly placed inside).
2) Afterwards, the software will indicate that it is ready for receiving samples. Remove the collecting vial from the heating chamber and add 5 μL to the first measurement vial (A-blue colour tap).
3) Use a similar procedure for the next two measurement vials, following the order: B-green colour tap and C-black colour tap.
4) Following this procedure, the user must tap the vials/cuvettes and invert it one time, to help homogenize the sample. Please wait 5 minutes and then insert each measurement vial on the system chamber/s, using the same cadency (A, B, C). This system will automatically ask for each one, using this order, to avoid user errors. Upon the three measurements the result will appear on the screen and stored into the system computer. At this time the end-user can chose if the data will be transmitted directly to the patient and the national health care entities.
5) A result is considered positive when two of the genetic sequences are detected on the sample (A, B, C) and negative when two are not present. This is automatically processed by the system and only the final result appears to the end-user. Nonetheless the complete data set will be gathered and stored.

Since the biosensing process needs to follow certain steps, the user will not only have access to a manual but also to an organigram of the biosensing procedure, with the steps and the respective order to achieve a result.

As will be clear to one skilled in the art, the present invention should not be limited to the embodiments described herein, and a number of changes are possible which remain within the scope of the present invention.

The disclosure should not be seen in any way restricted to the embodiments described and a person with ordinary skill in the art will foresee many possibilities to modifications thereof.

The above-described embodiments are combinable.

**Claims**

1. A system for fluorescence-based detection and quantification of a biomolecule in a sample, the system comprising three distinct units: a first unit comprising a heating unit with at least one integrated heat probe and a data integration system, such as a computer or an exterior computer; a second unit comprising at least one optical unit, preferably a spectrometer, which is connected to the computer or exterior computer of the first unit and means for receiving a sample containing the biomolecule to be analysed; and a third unit comprising a timer and a temperature control unit.

2. System according to the previous claim further comprising an oligonucleotide chosen from: SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9 and/, SEQ ID No. 10 SEQ ID No. 11, or SEQ ID No. 12.

3. System according to any of the previous claims, wherein said units comprise primarily iron, about 18-20% chromium and 8-10.5% (w/w) nickel and optionally small amounts, particularly $\leq$ 2.0% (w/w), of manganese, silicon, carbon and/or phosphorus.

4. System according to any of the previous claims further comprising an external power source, such as an electrical power supply unit, a battery module, a solar power unit, a USB power interface and/or a fuel cell unit, wherein the external power source system is configured to selectively or concurrently provide power from one or more of the power units to the system based on power availability, environmental conditions, or user preference.

5. System according to any of the previous claims further comprising an excitation source comprising a wavelength from 400 to 850 nm.

6. System according to any of the previous claims further comprising an excitation source, such as a LED, a laser, a xenon lamp, a mercury vapor lamp and/or a halogen lamp.

7. System according to any of the previous claims further comprising means for centrifugation, particularly suitable to centrifugate samples at a maximum speed from 6000 rpm to 12000 rpm, which is incorporated inside the system or used externally.

8. System according to any of the previous claims further comprising means for pre-treatment of the sample, preferably centrifugation means and/or agitation means, such as vortex.

9. System according to any of the previous claims further comprising at least one optical fiber with quartz core, a sampler unit, a transistor for LED, a pressure switch for the heating unit

10. System comprising the system of any of the previous claims enclosed in a case, such as a box.

11. Process for *in vitro* detecting a given biomolecule in the system of any of the previous claims comprising the steps of:

recognizing a given fluorescent-based diagnostic kit;
collecting a sample, preferably a nasopharyngeal or oropharyngeal swab, serum, blood, faeces or a tissue extract;
optionally pre-treating said sample;
placing the sample in a vial, preferably a tube, comprising an inactivation solution;
assigning a code bar to said sample;
passing said sample through the bar code reader or QR code identification unit;
placing said sample, preferably 5$\mu$l, into one to three vials/cuvettes;
detecting said biomolecule in said sample by identifying a target genetic sequence.

12. Use of the system of claims 1-9 in medicine, preferably in *in-vitro* cancer detection and monitoring, infectious disease diagnosis, genetic disorders screening, pharmacogenomics for personalized medicine and neurological disorder diagnosis.

13. Use of the system of claims 1-9 for *in-vitro* detection of *Eutypa lata,* HEV, SARS-CoV-2 infection or Grapvine **Trunk** Diseases.

B - External biosensor box

A - Interior supporting units

Fig. 1

A - Interior supporting units

Fig. 2

Fig. 3

**Fig. 4**

**Fig. 5**

1- HCV+
2- HBV+
3- HAV+
4- HAV+
5-CMV+
6- CMV+
7- EBV-+
8- HEV+
9- HEV+

**Fig. 6**

**Fig. 7**

**Fig. 8**

Fig. 9

a)

DL= 61±2 pM
QL= 186±1 pM
Sen.= 6.1 ± 0.3 (x10$^{11}$) pM/ a.u.

b)

DL= 82±3 pM
QL= 248±2 pM
Sen.= 5.1 ± 0.2 (x10$^{11}$) pM/ a.u.

c)

DL= 61±2 pM
QL= 185±1 pM
Sen.= 5.4 ± 0.3 (x10$^{11}$) pM/ a.u.

Fig. 10

Fig. 11

Fig. 12

Fig. 13

a)

b)

c)

d1)

d2)

e)

f)

g1)

g2)

h)

i)

j)

Fig. 14

| | Europäisches Patentamt / European Patent Office / Office européen des brevets | **EUROPEAN SEARCH REPORT** | **Application Number** EP 25 16 7781 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/007298 A1 (UNIV DE TRAS OS MONTES E ALTO DOURO [PT] ET AL.) 2 February 2023 (2023-02-02) | 1-10,12, 13 | INV. G01N33/50 G01N33/58 C12Q1/04 C12Q1/6816 C12Q1/6841 |
| Y | * (see par. [001], [014], [016]-[038], [042]-[043], Fig. 2 * | 11 | |
| X | WO 2021/220192 A2 (STAB VIDA INVESTIG E SERVICOS EM CIENCIAS BIOLOGICAS LDA [PT]) 4 November 2021 (2021-11-04) | 1-10,12, 13 | |
| Y | * see p. 12-18, 23-28, Fig. 1 * | 11 | |
| A | VINOTH KUMAR RAJENDRAN ET AL: "Smartphone technology facilitates point-of-care nucleic acid diagnosis: a beginner's guide", CRITICAL REVIEWS IN CLINICAL LABORATORY SCIENCES., 1 July 2020 (2020-07-01), pages 1-24, XP055712694, US ISSN: 1040-8363, DOI: 10.1080/10408363.2020.1781779 * the whole document * | 1-13 | |
| A | MAHANI MOHAMAD ET AL: "A carbon dot and molecular beacon based fluorometric sensor for the cancer marker microRNA-21", MICROCHIMICA ACTA, vol. 186, no. 3, 1 February 2019 (2019-02-01), XP093306978, Vienna ISSN: 0026-3672, DOI: 10.1007/s00604-019-3233-z Retrieved from the Internet: URL:http://link.springer.com/article/10.10 07/s00604-019-3233-z/fulltext.html> * the whole document * | 1-13 | |

**TECHNICAL FIELDS SEARCHED (IPC)**

C12Q
G01N

-/--

The present search report has been drawn up for all claims

| Place of search | Date of completion of the search | Examiner |
|---|---|---|
| The Hague | 27 August 2025 | Diez Schlereth, D |

EPO FORM 1503 03.82 (P04C01)

Europäisches
Patentamt

European
Patent Office

Office européen
des brevets

# EUROPEAN SEARCH REPORT

**Application Number**

EP 25 16 7781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FUJIYOSHI PHILLIP T. ET AL: "DNA-based detection of grapevine trunk-disease pathogens from environmental spore samples", METHODSX, vol. 8, 1 January 2021 (2021-01-01), page 101494, XP093306980, NL ISSN: 2215-0161, DOI: 10.1016/j.mex.2021.101494 * the whole document * | 1-13 | |

----- 

**TECHNICAL FIELDS
SEARCHED       (IPC)**

The present search report has been drawn up for all claims

| Place of search | Date of completion of the search | Examiner |
|---|---|---|
| The Hague | 27 August 2025 | Diez Schlereth, D |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

.............................................................................................
& : member of the same patent family, corresponding
 document

EPO FORM 1503 03.82 (P04C01)

page 2 of 2

## ANNEX TO THE EUROPEAN SEARCH REPORT
## ON EUROPEAN PATENT APPLICATION NO.

EP 25 16 7781

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2025

| Patent document cited in search report | Publication date | Patent family member(s) | | Publication date |
|---|---|---|---|---|
| WO 2023007298 A1 | 02-02-2023 | NONE | | |
| WO 2021220192 A2 | 04-11-2021 | AU 2021265579 | A1 | 10-11-2022 |
| | | BR 112022021086 | A2 | 13-12-2022 |
| | | CA 3175648 | A1 | 04-11-2021 |
| | | CN 115836133 | A | 21-03-2023 |
| | | EP 4142945 | A2 | 08-03-2023 |
| | | IL 297281 | A | 01-12-2022 |
| | | JP 2023524117 | A | 08-06-2023 |
| | | WO 2021220192 | A2 | 04-11-2021 |

EPO FORM P0459

For more details about this annex : see Official Journal of the European Patent Office, No. 12/82